# EUROPEAN PATENT APPLICATION

(11) **EP 4 635 414 A1**
(43) Date of publication of application: **22.10.2025**
(21) Application number: 23902259.3
(22) Date of filing: 11.10.2023
(51) Int. Cl.: A61B 5/145

(54) **SENSOR ASSEMBLY, NEEDLE BOX CONTAINING SENSOR ASSEMBLY, AND DEDICATED PAINLESS PATCH**

(30) Priority: 12.12.2022 CN 202211597534
(71) Applicant: Teljane Medical Technology (Suzhou) Co., Ltd, Suzhou, Jiangsu 215000 (CN)
(72) Inventor: MAO, Jian, Suzhou, Jiangsu 215000 (CN); TANG, Rongjun, Suzhou, Jiangsu 215000 (CN); LIU, Feifei, Suzhou, Jiangsu 215000 (CN)
(74) Representative: Haseltine Lake Kempner LLP
(86) International application number: PCT/CN2023/123913
(87) International publication number: WO 2024/125057

(57) **Abstract**

Provided is a sensor assembly, including sensor electrode, a sensor waterproof soft rubber, a plastic base and a rigid needle. The sensor electrode is formed by a horizontal connecting part and a vertical implanting part which are connected to form an L-shaped structure, thus preventing the sensor electrode from breaking after implementation into a human body. The sensor electrode is in lateral contact with the conductive silicone elements, featuring a secure and stable connection, resistance to shaking and good data stability. Further provided is a needle box, containing the sensor assembly. The needle box may reliably store the sensor assembly and is convenient to use. Further provided is a dedicated painless patch applicator, which has a few of parts and a simple structure, is easy to assemble, realizes part assembly without a hot-melting process, avoids false triggering, realizes needle insertion by means of one spiral spring to make the whole insertion process faster, and minimizes pains to realize painless needle insertion.

## Description

### Field of the Invention

The present invention relates to a sensor assembly, a needle box containing the sensor assembly, and a dedicated painless patch applicator.

### Background of the Invention

It is very important for diabetic patients to monitor their blood glucose, and the blood glucose level is used for assessing glucose metabolic disorders of the diabetic patients. At present, the blood glucose is detected in vitro based on blood sampling or is detected in real time by means of implantable blood glucose sensors. When the in-vitro detection method based on blood sampling is used for blood glucose control, the blood has to be sampled multiple times every day, bringing a heavy metal and physical burden to patients; in addition, limited by the times of measurement, only the blood glucose level at the measurement moment may be provided, and fluctuations of the blood glucose level within a day cannot be completely reflected. Meanwhile, limited by the times of measurement, the variation trend and direction of the measured blood glucose level cannot be determined, so hypoglycemia is more likely to occur by adjusting the dosage of insulin according to the measured blood glucose level. This problem may be effectively solved by dynamic blood glucose detection. A dynamic blood glucose monitoring system not only can provide the blood glucose level in real time, but also can display the variation trend of the blood glucose level, and can more accurately determine the variation and trend of the blood glucose level in conjunction with daily routines of patients. Thus, real-time detection methods based on implantable blood glucose sensors are becoming increasingly popular.

At present, blood glucose continuous-monitoring products, that are suitable for clinical use and technically verified to some extent, are sensor assemblies implanted into hypodermis. **In** the art, it is necessary to implant a flexible detection part of a tiny and soft biosensor into skin and insert a puncture tube into the skin extremely quickly to minimize pains and to retain an electrode part of the sensor in the skin to realize continuous monitoring, with a part, outside the skin, of the sensor being fixed to the skin.

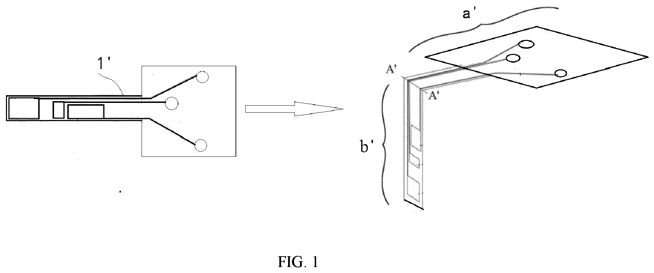

Referring to FIG. 1, at present, a sensor electrode 1' of a sensor in glucose monitoring devices sold on the market is generally manufactured by screen printing and configured a strip-shaped planar structure. When installed, the sensor electrode 1' needs to be bent by a certain angle (about 90°), such that the sensor electrode 1' is divided into an implanting part b' implanted into the human body and a connecting part a' connected to an electronic system. After such a sensor electrode is worn, a bent part A'-A' formed during installation has an impact on various coatings on the sensor electrode and is prone to breakage in the implantation and wearing process; the connecting part a' is externally connected to the electronic system by means of a conductive contact crimped thereon, the conductive contact is flatly crimped on an electrode of the connecting part a', and if the conductive contact is crimped insecurely, the implanting part will shake in the human body, compromising wearing comfort, leading to loud data background noise and affecting data stability.

**In** addition, the blood glucose continuous-monitoring product typically includes an insertion aid, a sensor and a transmitter. The transmitter is generally mounted in the insertion aid. **In** use, the insertion aid provided with the transmitter and the sensor are assembled together by users, and the insertion aid, the sensor and the transmitter are sold as a set of equipment and are disposable, leading to a high cost. The insertion aid is used for implanting a soft detection part of the sensor into skin. The insertion aid in the prior art has a complex part structure and generally adopts two springs to complete the whole needle insertion process, wherein the precompression potential energy of one spring is used for needle insertion, and the precompression potential energy of the other spring is used for needle withdrawal. Or, a rigid needle and a soft needle are inserted into the human body with manpower. Specifically, users press the whole insertion aid to implant the detection part of the sensor into skin with large strength and withdraws the needles by means of a withdrawing mechanism in the insertion aid. **In** use, the users often feel uncomfortable due to excessively large or small strength; two mechanisms are used for needle insertion and needle withdrawal respectively; in the needle insertion process, the rigid needle is retained in the human body for about 40-50 ms, the retention time of the rigid needle in the human body is too long, and pains will be caused when the rigid needle is implanted. Moreover, if the users mistouch a start button of the insertion aid in use, the insertion aid will be directly started, leading to a failure of the whole blood glucose continuous-monitoring product.

### Summary of the Invention

One objective of the present invention is to overcome the defects in the prior art by providing a sensor assembly provided with a sensor electrode, which is directly designed into an L-shaped structure to be prevented from breaking after being implanted into a human body and forms a secure lateral connection with the conductive silicone, ensuring stability and resistance to shaking and good data stability.

Another objective of the present invention is to provide a needle box containing the sensor assembly. The needle box may stably store the sensor assembly and is convenient to use.

Another objective of the present invention is to provide a dedicated painless patch applicator, which has a few of parts and a simple structure, is easy to assemble, avoids false triggering and makes the whole needle insertion process more stable and reliable; and needle insertion is realized by one spiral spring, the whole needle insertion process is faster, and pains are minimized, thereby realizing painless needle insertion.

One technical solution adopted to fulfill the objectives is as follows: a sensor assembly includes a sensor electrode, a sensor waterproof soft rubber, a plastic base and a rigid needle, wherein:
the sensor electrode is formed by a horizontal connecting part and a vertical implanting part which are connected to form an L-shaped structure, the horizontal connecting part is provided with three electrodes, and the three electrodes are located on front and back sides of the horizontal connecting parts, respectively;
three conductive silicone mounting holes are formed in a top end of the sensor waterproof soft rubber, the three conductive silicone mounting holes are distributed in a triangular shape, and a conductive silicone is arranged in each of the conductive silicone mounting holes;
a strip-shaped sensor electrode channel is formed inwards in a side wall of the sensor waterproof soft rubber, and the sensor electrode channel is located among the three conductive silicone mounting holes and communicates with the three conductive silicone mounting holes;
the horizontal connecting part of the sensor electrode is inserted into the sensor electrode channel, and the three electrodes on the horizontal connecting part are in interference fit and contact with side surfaces of the three the conductive silicone elements in one-to-one correspondence;
a waterproof soft rubber mounting groove and a vertically through rigid needle hole are formed in the plastic base;
the sensor waterproof soft rubber is arranged in the waterproof soft rubber mounting groove, and the vertical implanting part of the sensor electrode is inserted into the rigid needle hole;
a notch is vertically formed in a side wall of the rigid needle, and the rigid needle penetrates through the rigid needle hole and is disposed around the vertical implanting part of the sensor electrode;
a rigid needle holder is arranged at a top end of the rigid needle, and the rigid needle holder is located above the rigid needle hole.

According to the sensor assembly, wherein joints between a tip of the rigid needle and two side walls of the notch are arc-shaped.

Another technical solution adopted to fulfill the above objectives is as follows: a needle box includes a needle box bottom shell, a needle box top shell and the sensor assembly, wherein:
a push piece is vertically arranged on an inner wall of the needle box bottom shell, and a rigid needle receiving groove is formed in the needle box bottom shell;
the needle box top shell is arranged in the needle box bottom shell, and a vertically through sensor assembly mounting hole is formed in the needle box top shell;
the sensor assembly is arranged in the sensor assembly mounting hole, and a lower portion of the rigid needle of the sensor assembly is inserted into the rigid needle receiving groove.

According to the needle box, wherein a sealing aluminum foil is heat-sealed over the opening in an upper end of the needle box bottom shell.

A third technical solution adopted to fulfill the above objectives is as follows: a dedicated painless patch applicator is used for pushing the sensor assembly in the needle box and implanting the vertical implanting part of the sensor electrode of the sensor assembly into human skin. The dedicated painless patch applicator includes an insertion aid and a transmitter assembly arranged in the insertion aid. The insertion aid includes a shell, a spiral spring, a turnplate, a button, an unlocking push rod, a sliding rail and a transmitter holder, wherein:
the shell is a hollow cavity structure with an opening in a lower end, formed by a Shell A and a Shell B;
a circular spring mounting groove, a rail sliding groove and a push rod sliding groove are formed in an inner wall of the Shell A, an axial direction of the circular spring mounting groove is perpendicular to an axial direction of the shell, and a stationary shaft is coaxially arranged in the circular spring mounting groove; the rail sliding groove is formed by two vertical rail sliding grooves, and the two vertical rail sliding grooves are arranged on two radial sides of the circular spring mounting groove, respectively; the push rod sliding groove is arranged vertically, and the push rod sliding groove is located between the circular spring mounting groove and one of the vertical rail sliding grooves;
a button mounting groove is formed in an outer wall of the Shell A, and a push rod sliding hole is formed in a side wall of the Shell A;
the spiral spring is mounted in the circular spring mounting groove and disposed around the stationary shaft; the turnplate is rotatably disposed around the stationary shaft, and a ring-shaped peripheral wall of the turnplate is located between the spiral spring and a wall of the circular spring mounting groove; an inner end of the spiral spring is connected to the stationary shaft; an outer end of the spiral spring is connected to the ring-shaped peripheral wall of the turnplate;
a turnplate bump is arranged on an outer surface of the ring-shaped peripheral wall of the tumplate, and a turnplate stop is arranged at a bottom of the turnplate;
the button is movably arranged in the button mounting groove of the Shell A, and a rear end of the button penetrates through a bottom of the button mounting groove to extend into the Shell A; a button bump is arranged on an inner wall of the rear end of the button, and in an initial state, the turnplate bump abuts against the button bump;
a front end of the unlocking push rod is inserted into the push rod sliding groove and slides along the push rod sliding groove; one side of the unlocking push rod is inserted into the push rod sliding hole and slides along the push rod sliding hole, and in the initial state, the rear end of the button abuts against the other side of the unlocking push rod;
a turnplate limiting groove is horizontally formed in a front side of an upper portion of the sliding rail, two sides of the sliding rail are inserted into the two vertical rail sliding grooves in one-to-one correspondence and slide along the corresponding vertical rail sliding grooves, and the turnplate stop is inserted into the turnplate limiting groove;
the transmitter holder is coaxially arranged in the shell;
a top end of the transmitter holder is connected to a bottom end of the sliding rail;
the transmitter assembly is arranged in the transmitter holder;
a rigid needle mounting hole is formed in the bottom end of the sliding rail, and a vertically through rigid needle hole is formed in the top end of the transmitter holder.

According to the dedicated painless patch applicator, wherein the transmitter assembly includes a transmitter housing and a transmitter circuit board assembly arranged in the transmitter housing, wherein:
the transmitter housing is formed by an upper housing and a lower housing which are connected together, and a transmitter bonding adhesive is arranged on a bottom surface of the transmitter housing; a sensor assembly mounting position is arranged on the lower housing of the transmitter housing;
the transmitter circuit board includes a transmitter circuit board, a Mylar Film, a button cell, a positive nickel sheet and a negative nickel sheet, and the Mylar Film is arranged at a bottom end of the transmitter circuit board; the button cell is located at a bottom end of the Mylar Film; the negative nickel sheet is welded to a negative pad of the transmitter circuit board; the positive nickel sheet is welded to a positive pad of the transmitter circuit board, and the positive nickel sheet and the negative nickel sheet are connected to the button cell;
rigid needle holder holes are formed in the upper housing of the transmitter housing and the transmitter circuit board, respectively;
a sensor assembly hole is formed in the transmitter bonding adhesive.

According to the dedicated painless patch applicator, wherein when the dedicated painless patch applicator is connected to the sensor assembly in the needle box, the sensor assembly penetrates through the sensor assembly hole in the transmitter bonding adhesive and is then inserted into the sensor assembly mounting position on the lower housing of the transmitter housing;
the rigid needle holder sequentially penetrates through the rigid needle holder hole in the transmitter circuit board, the rigid needle holder hole in the upper housing of the transmitter housing and the rigid needle hole in the top end of the transmitter holder and is then clamped in the rigid needle mounting hole in the bottom end of the sliding rail.

According to the dedicated painless patch applicator, wherein when the dedicated painless patch applicator is connected to the sensor assembly in the needle box, the push piece on the inner wall of the needle box bottom shell pushes the side, inserted into the push rod sliding hole, of the unlocking push rod to slide upwards to drive the whole unlocking push rod to slide upwards.

According to the dedicated painless patch applicator, wherein a lower portion of the shell is covered with a bottom cover.

According to the dedicated painless patch applicator, wherein a transmitter holder stop is arranged at a bottom end of the inner wall of the shell, and when the insertion aid is withdrawn after use, the transmitter holder is clamped by the transmitter holder stop.

According to the dedicated painless patch applicator, wherein a downward indicating arrow is arranged on a side wall of the shell and is adjacent to the push rod sliding hole; and an indicating groove is formed in an upper end of the needle box bottom shell, and the indicating groove is adjacent to the push piece.

By adopting the technical solutions of the sensor assembly, the needle box containing the sensor assembly and the dedicated painless patch applicator, the sensor electrode in the sensor assembly is directly designed into the L-shaped structure, thereby not being prone to breakage after being implanted into a human body; and the sensor electrode is in lateral contact with the the conductive silicone elements, featuring a firm and stable connection, resistance to shaking and good data stability. The needle box may reliably store the sensor assembly and is convenient to use. The dedicated painless patch applicator has a few of parts and a simple structure, is easy to assemble, avoids false triggering and makes the whole insertion process more stable and reliable; and needle insertion is realized by one spiral spring, the whole needle insertion process is faster, and pains are minimized, thereby realizing painless needle insertion.

### Brief Description of the Drawings

FIG. 1 is a schematic structural diagram of a sensor electrode of a glucose monitoring device in the prior art;
FIG. 2 is a stereogram of a sensor assembly according to the present invention;
FIG. 3 is a schematic structural diagram of a sensor electrode of the sensor assembly according to the present invention;
FIG. 4 is a schematic structural diagram of a needle box according to the present invention;
FIG. 5 is an exploded structural view of the needle box according to the present invention;
FIG. 6 is a sectional view of the needle box according to the present invention;
FIG. 7 is a top view of the needle box according to the present invention;
FIG. 8 is a front view of a dedicated painless patch applicator according to the present invention;
FIG. 9 is an exploded structural view of the dedicated painless patch applicator according to the present invention;
FIG. 10 is an internal structural diagram of the dedicated painless patch applicator according to the present invention;
FIG. 11 is a sectional view of the dedicated painless patch applicator according to the present invention;
FIG. 12 is a schematic installation diagram of a spiral spring of the dedicated painless patch applicator according to the present invention;
FIG. 13 is a schematic installation diagram of a turnplate of the dedicated painless patch applicator according to the present invention;
FIG. 14 is a schematic installation diagram of an unlocking push rod of the dedicated painless patch applicator according to the present invention;
FIG. 15 is a schematic limiting diagram of the unlocking push rod and a button;
FIG. 16 is a schematic installation diagram of a sliding rail of the dedicated painless patch applicator according to the present invention;
FIG. 17 is a schematic diagram of the connection process of the sliding rail, a transmitter holder and a transmitter assembly;
FIG. 18 is a schematic structural diagram of the transmitter assembly;
FIG. 19 is a schematic structural diagram of a transmitter circuit board assembly;
FIG. 20 is an exploded structural view of the transmitter circuit board assembly;
FIG. 21 is a schematic connection diagram of the dedicated painless patch applicator and the sensor assembly in the needle box;
FIG. 22 is a sectional view of FIG. 21;
FIG. 23 is a diagram of the working principle of the dedicated painless patch applicator according to the present invention;
FIG. 24 is a comparison diagram of a case where a turnplate stop of the turnplate moves in a strip-shaped turnplate limiting groove and a case where the turnplate stop of the turnplate moves in an arc-shaped turnplate limiting groove (under the same rotation angle of the turnplate);
FIG. 25 is a comparison diagram of the case where the turnplate stop of the turnplate moves in the strip-shaped turnplate limiting groove and the case where the turnplate stop of the turnplate moves in the arc-shaped turnplate limiting groove (under the same sliding distance of the turnplate);
FIG. 26 is a principle diagram for detecting the retention time of a rigid needle in a human body.

### Detailed Description of the Invention

To allow those skilled in the art to gain a better understanding of the technical solutions of the present invention, specific embodiments of the present invention are described in detail below in conjunction with accompanying drawings.

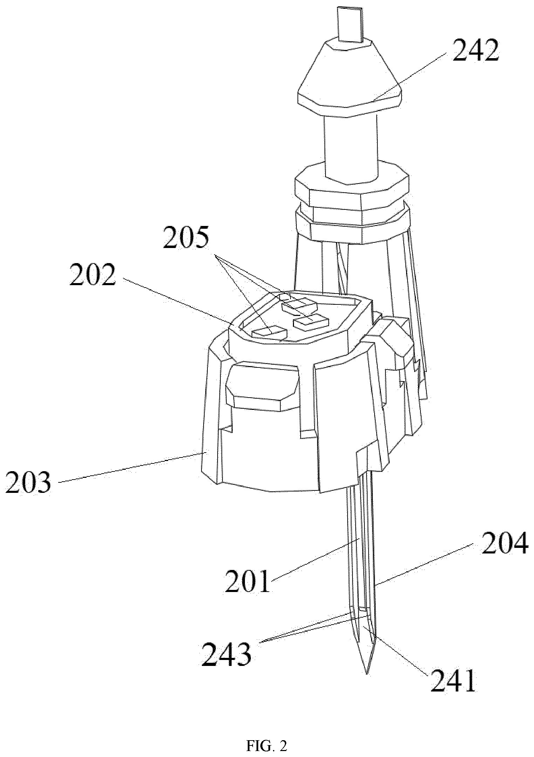

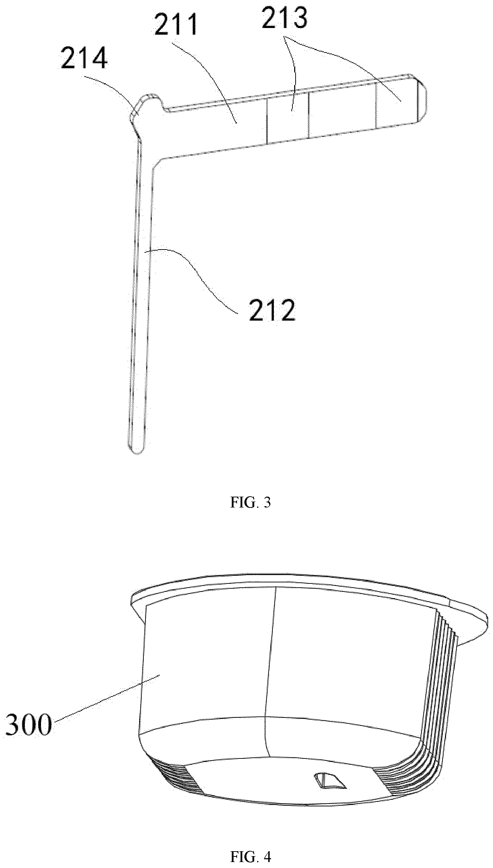

Referring to FIGS. 2 and 3, an embodiment of the present invention provides a sensor assembly, including a sensor electrode 201, a sensor waterproof soft rubber 202, a plastic base 203 and a rigid needle 204.

The sensor electrode 201 is formed by a horizontal connecting part 211 and a vertical implanting part 212 which are connected to from an L-shaped structure. The horizontal connecting part 211 is provided with three electrodes 213, and the three electrodes 213 are located on front and back sides of the horizontal connecting part 211, respectively. A bump 214 protruding upwards is arranged at a top end of a joint between the horizontal connecting part 211 and the vertical implanting part 212. The three electrodes are a reference electrode, a working electrode and an auxiliary electrode, respectively. Two of the three electrodes are arranged on the front side of the horizontal connecting part 211 of the sensor electrode 201, and the other electrode is arranged on the back side of the horizontal connecting part 211 of the sensor electrode 201.

Three conductive silicone mounting holes are formed in a top end of the sensor waterproof soft rubber 202 and distributed in a triangular shape, and a conductive silicone 205 is arranged in each of the conductive silicone mounting holes. A strip-shaped sensor electrode channel is formed inwards in a side wall of the sensor waterproof soft rubber 202, and the sensor electrode channel is located among the three conductive silicone mounting holes and communicates with the three conductive silicone mounting holes. The horizontal connecting part 211 of the sensor electrode 201 is inserted into the sensor electrode channel, and the three electrodes 213 on the horizontal connecting part 211 are in interference fit and contact with side surfaces of the three the conductive silicone elements 205 in one-to-one correspondence.

A waterproof soft rubber mounting groove and a vertically through rigid needle hole are formed in the plastic base 203. The sensor waterproof soft rubber 202 is arranged in the waterproof soft rubber mounting groove, and the vertical implanting part 212 of the sensor electrode 201 is inserted into the rigid needle hole. A notch 241 is vertically formed in a side wall of the rigid needle 204, and the rigid needle 204 penetrates through the rigid needle hole and is disposed around the vertical implanting part 212 of the sensor electrode 201. A rigid needle holder 242 is arranged at a top end of the rigid needle 204 and located above the rigid needle hole. Joints 243 between a tip of the rigid needle 204 and two side walls of the notch 241 are arc-shaped. Compared a traditional rigid needle structure with sharp corners being formed at joints 243 between a tip of a traditional rigid needle 204 and two side walls of a notch 241, the arc-shaped design in the present invention may reduce resistance and puncture force to relieve pains in the puncture process when the rigid needle 204 is inserted into a human body together with the vertical implanting part 212 of the sensor electrode 201.

When the sensor assembly provided by the present invention is assembled, first, the sensor electrode 201 is assembled in the sensor waterproof soft rubber 202; then, the three the conductive silicone elements 205 are assembled in the sensor waterproof soft rubber 202; next, a steel needle is disposed around the vertical implanting part 212 of the sensor electrode 201; and finally, the steel needle and the sensor waterproof soft rubber 202 are synchronously assembled in the plastic base 203. After the sensor assembly is assembled, whether all parts are assembled in place is checked.

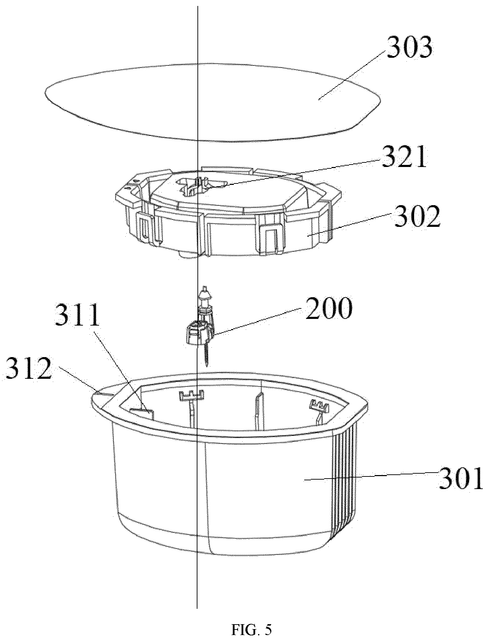

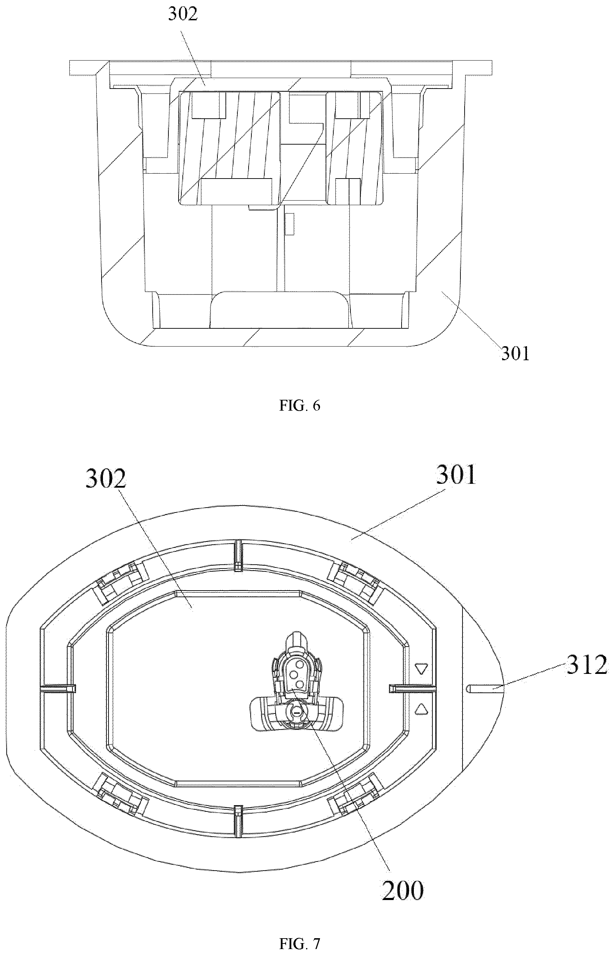

Referring to FIGS. 4, 5, 6 and 7, a needle box 300 includes a needle box bottom shell 301, a needle box top shell 302 and the sensor assembly 200. A push piece 311 is vertically arranged on an inner wall of the needle box bottom shell 301. A rigid needle receiving groove is formed in the needle box bottom shell 301. The needle box top shell 302 is arranged in the needle box bottom shell 301, and a vertically through sensor assembly mounting hole 321 is formed in the needle box top shell 302.

The sensor assembly 200 is arranged in the sensor assembly mounting hole 321, and a lower portion of the rigid needle 204 of the sensor assembly 200 is inserted into the rigid needle receiving groove. A sealing aluminum foil 303 is heat-sealed over the opening in an upper end of the needle box bottom shell 301. An indicating groove 312 is formed in the upper end of the needle box bottom shell 301 and is adjacent to the push piece 311.

When the needle box is assembled, first, the sensor assembly is assembled in the needle box bottom shell; then, the needle box top shell is assembled in the needle box bottom shell, and whether the steel needle, the needle box top shell and other parts are assembled in place is checked; and the sealing aluminum foil 303 is heat-sealed over the opening in the upper end of the needle box bottom shell.

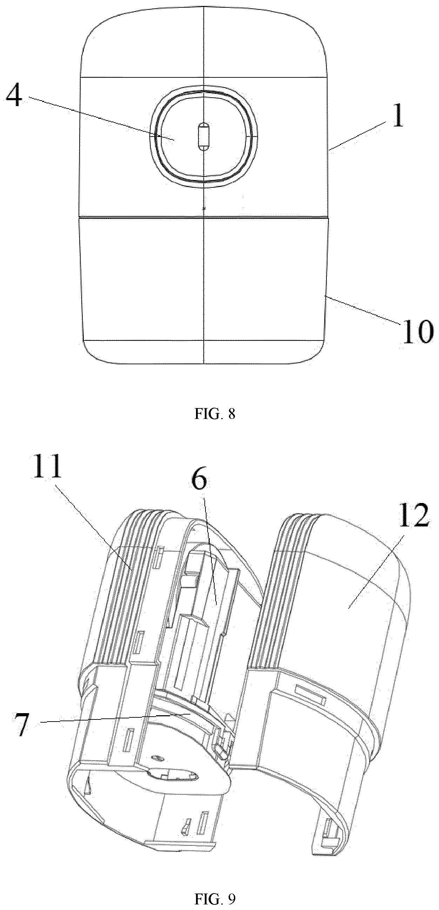

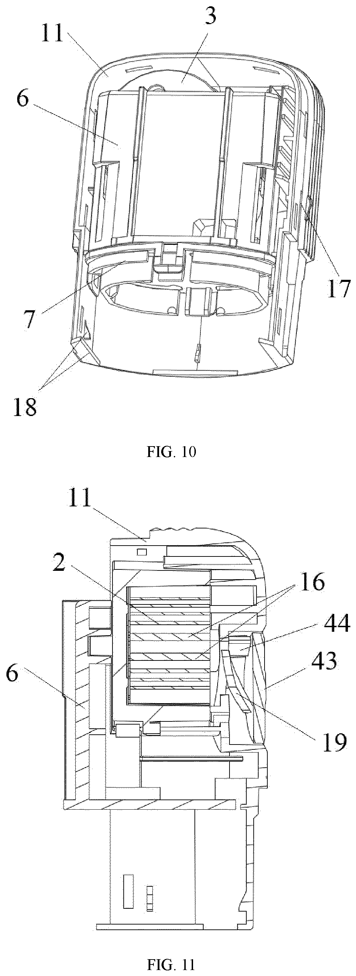

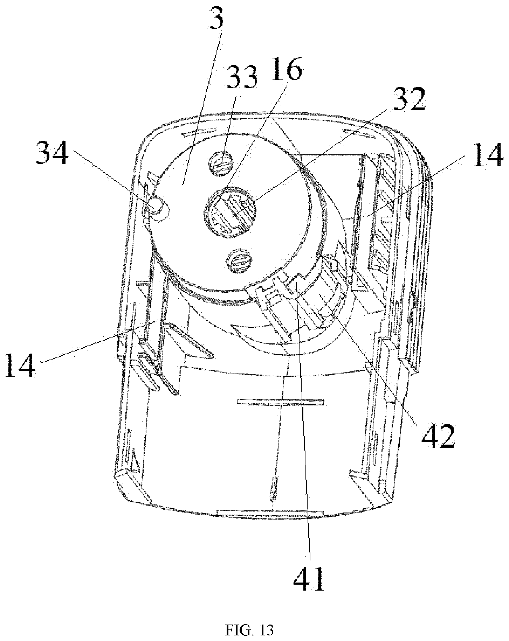

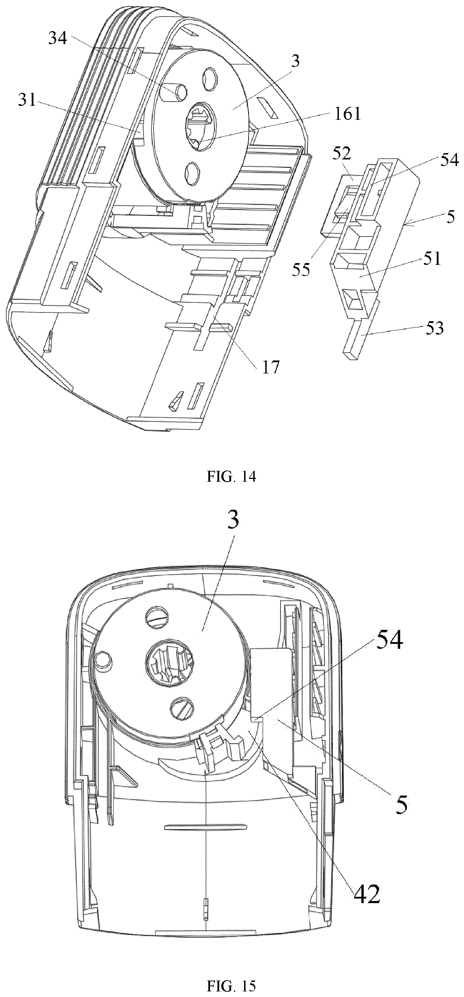

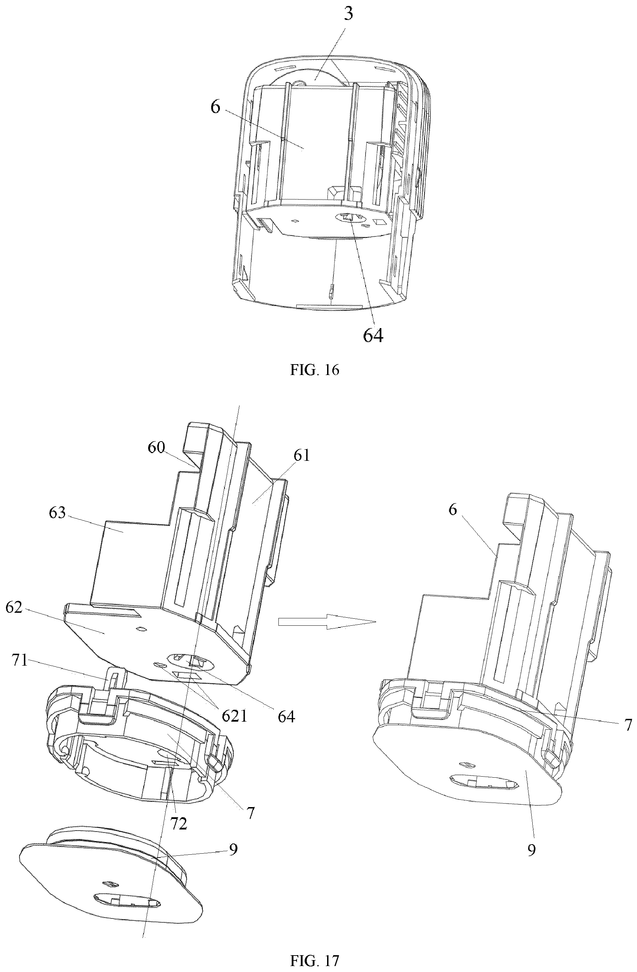

Referring to FIGS. 8-17, a dedicated painless patch applicator is used for pushing the sensor assembly 200 in the needle box 300 and implanting the vertical implanting part 212 of the sensor electrode 201 of the sensor assembly 200 into human skin. The dedicated painless patch applicator includes an insertion aid and a transmitter assembly 9 arranged in the insertion aid. The insertion aid includes a shell 1, a spiral spring 2, a turnplate 3, a button 4, an unlocking push rod 5, a sliding rail 6 and a transmitter holder 7.

The shell 1 is a hollow cavity structure with an opening in a lower end, formed by a Shell A 11 and a Shell B 12. A circular spring mounting groove 13, a rail sliding groove and a push rod sliding groove 15 are formed in an inner wall of the Shell A 11. An axial direction of the circular spring mounting groove 13 is perpendicular to an axial direction of the shell 1, and a stationary shaft 16 is coaxially arranged in the circular spring mounting groove 13. The rail sliding groove is formed by two vertical rail sliding grooves 14, and the two vertical rail sliding grooves 14 are located on two radial sides of the circular spring mounting groove 13, respectively. The push rod sliding groove 15 is arranged vertically and located between the circular spring mounting groove 13 and one of the vertical rail sliding grooves 14.

A button mounting groove is formed in an outer wall of the Shell A 11, and a push rod sliding hole 17 is formed in a side wall of the Shell A 11.

The spiral spring 2 is mounted in the circular spring mounting groove 13 and disposed around the stationary shaft 16. The turnplate 3 is rotatably disposed around the stationary shaft 16, and a ring-shaped peripheral wall of the turnplate 3 is located between the spiral spring 2 and a wall of the circular spring mounting groove 13. An inner end of the spiral spring 2 is connected to the stationary shaft 16. An outer end of the spiral spring 2 is connected to the ring-shaped peripheral wall of the turnplate 3. Specifically, the stationary shaft 16 is cylindrical, a spring inner end socket 161 is formed in a wall of the stationary shaft 16 in an axial direction of the stationary shaft 16, and a spring outer end socket 31 is formed in the ring-shaped peripheral wall of the turnplate 3 in an axial direction of the turnplate 3. The inner end of the spiral spring 2 is clamped in the spring inner end socket 161, and the outer end of the spiral spring 2 is clamped in the spring outer end socket 31. Precompression potential energy of the spiral spring 2 pushes the turnplate 3 to rotate.

A shaft hole 32 and two rotation holes 33 are formed in the bottom of the turnplate 3, the two rotation holes 33 are located in two radial sides of the shaft hole 32, and the stationary shaft 16 is inserted into the shaft hole 32 in the bottom of the turnplate. During assembly, two bumps of a turnplate rotating tool are clamped in the two rotation holes 33, and the turnplate 3 may be rotated to drive the spiral spring 2 to rotate to enable the precompression potential energy of the spiral spring 2 to reach a suitable intensity to realize needle insertion and needle withdrawal.

A turnplate bump is arranged on an outer surface of the ring-shaped peripheral wall of the turnplate 3, and a turnplate stop 34 is arranged at the bottom of the turnplate 3.

The button 4 is movably arranged in the button mounting groove in the Shell A 11, a rear end of the button 4 penetrates through the bottom of the button mounting groove to extend into the Shell A 11. A button bump 41 is arranged on an inner wall of the rear end of the button 4, and in an initial state, the turnplate bump abuts against the button bump 41.

Specifically, the button mounting groove is located on a front side of the circular spring mounting groove 13, and the bottom of the button mounting groove is partially overlapped with the bottom of the circular spring mounting groove 13. Two button holes are formed in the bottom of the button mounting groove. The button 4 includes a button body 43, a short button pole 44 and a long button pole 42, and the short button pole and the long button pole are arranged at a rear end of the button body 43. The short button pole 44 and the long button pole 42 penetrate through the two button holes in one-to-one correspondence, and the short button pole 43, after penetrating through the corresponding button hole, is located in the stationary shaft 16. The button bump 41 is located on an inner wall of a rear end of the long button pole 42. A button clip 19 protruding forwards is arranged at the bottom of the button mounting groove, and the button body 43 is in contact with the button clip 19. When the button 4 is pressed, the long button pole 42 drives the button bump 41 to move backwards until the turnplate bump is separated from the button bump 41, and at this moment, the turnplate 3 rotates under the action of the spiral spring 2.

A front end of the unlocking push rod 5 is inserted into the push rod sliding groove 15 and slides along the push rod sliding groove 15. One side of the unlocking push rod 5 is inserted into the push rod sliding hole 17 and slides along the push rod sliding hole 17. In the initial state, the rear end of the button 4 abuts against the other side of the unlocking push rod 5.

Specifically, the unlocking push rod 5 includes a push rod body 51, a front push rod insertion piece 52 arranged at a front end of the push rod body 51, a side push rod insertion piece 53 arranged on a lower portion of one side of the push rod body and a button abutting step 54 arranged on an upper portion of the other side of the push rod body. The front push rod insertion piece 52 is inserted into the push rod sliding groove 15 and slides along the push rod sliding groove 15. The side push rod insertion piece 53 is inserted into the push rod sliding hole 17 and slides along the push rod sliding hole 17. In the initial state, the rear end of the long button pole 42 of the button 4 abuts against a front side of the button abutting step 54, and at this moment, even if users mistouch the button 4, the button will not be pushed to move backwards under the abutting effect of the button abutting step 54 of the unlocking push rod 5. In use, a push piece is arranged on the needle box bottom shell where the sensor is assembled; when the painless patch applicator is connected to the sensor in the needle box bottom shell, the push piece pushes the side push rod insertion piece 53 to slide upwards along the push rod sliding hole 17, at the same time, the front push rod insertion piece 52 moves upwards along the push rod sliding groove 15 to fulfill a guide effect, the whole unlocking push rod 5 moves upwards, the rear end of the long button pole 42 of the button 4 is separated from the button abutting step 54, the button 4 is unlocked, and then the button 4 can be pressed backwards.

An elastic piece 55 is arranged on the front push rod insertion piece 52, and a corrugated strip 151 matched with the elastic piece 55 is arranged on a wall of the push rod sliding groove 15.

A turnplate limiting groove 60 is horizontally formed in a front side of an upper portion of the sliding rail 6, two sides of the sliding rail are inserted into the two vertical rail sliding grooves 14 in one-to-one correspondence and slide along the corresponding vertical rail sliding grooves, and the turnplate stop 34 is inserted into the turnplate limiting groove 60. When the turnplate 3 rotates, the turnplate stop 34 in the turnplate limiting groove 60 pushes the sliding rail 6 to move downwards and then move upwards.

The transmitter holder 7 is coaxially arranged in the shell 1, and a top end of the transmitter holder 7 is connected to a bottom end of the sliding rail 6.

Specifically, the sliding rail 6 includes a rear rail plate 61, a bottom rail plate 62 and two guide bars 63. The rear rail plate 61 and the bottom rail plate 62 are connected to form an L-shaped structure, and the two guide bars 63 are connected to two sides of the rear rail plate 61 in one-to-one correspondence. The turnplate limiting groove 60 is horizontally formed in an upper portion of a front side of the rear rail plate 61. The two guide bars 63 of the sliding rail 6 are inserted into the two vertical rail sliding grooves 14 in one-to-one correspondence and slide along the corresponding vertical rail sliding grooves.

A transmitter holder insertion slot 621 is formed in the bottom rail plate 62, a transmitter holder insertion piece 71 is arranged at the top end of the transmitter holder 7, and the transmitter holder insertion piece 71 is inserted into the transmitter holder insertion slot 621.

A rigid needle mounting hole 64 is formed in the bottom end of the sliding rail 6 (i.e., the bottom rail plate 62), and a vertically through rigid needle hole 72 is formed in the top end of the transmitter holder 7. When the painless patch applicator is connected to the sensor assembly in the needle box bottom shell, the top end of the rigid needle 204 on the sensor assembly penetrates through the rigid needle hole 72 and is then clamped in the rigid needle mounting hole 64; and when the sliding rail 6 moves upwards and downwards, the rigid needle 204 is driven to move upwards and downwards.

The transmitter holder 7 is configured as a slot structure with an opening formed in a lower end. A transmitter assembly 9 is arranged in the transmitter holder 7. A lower portion of the shell 1 is covered with a bottom cover 10. A transmitter holder stop 18 is arranged at a bottom end of the inner wall of the shell 1. When the insertion aid is withdrawn after use, the transmitter holder 7 is clamped by the transmitter holder stop 18 and retained in the shell 1 to be removed synchronously.

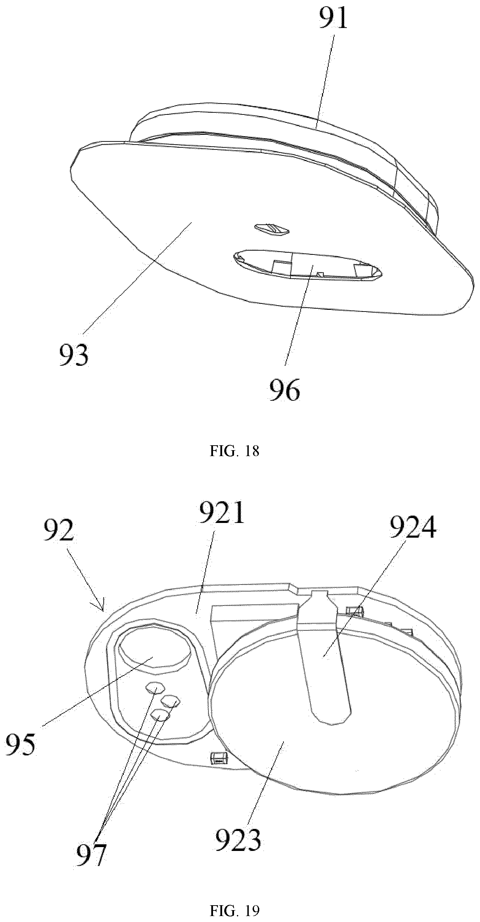

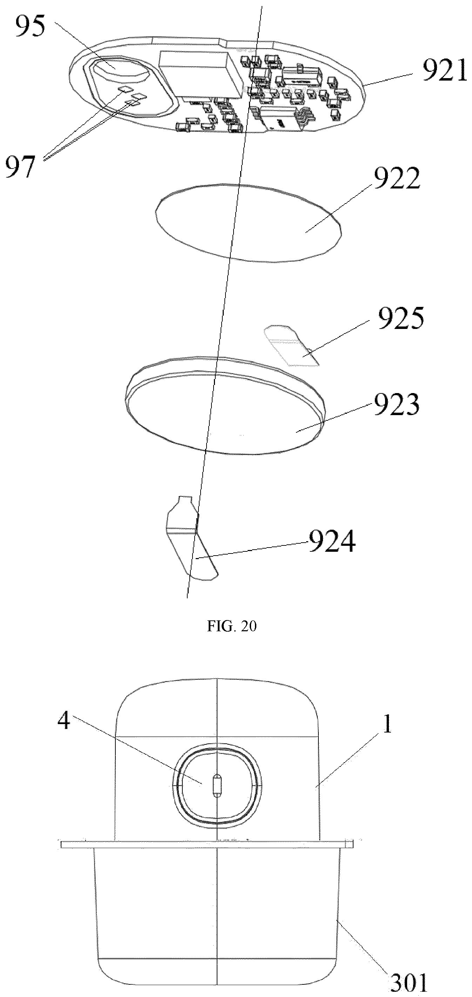

Referring to FIGS. 18, 19 and 20, the transmitter assembly 9 includes a transmitter housing 91 and a transmitter circuit board assembly 92 arranged in the transmitter housing 91, wherein the transmitter housing 91 is formed by an upper housing and a lower housing which are connected together. A transmitter bonding adhesive 93 is arranged on a bottom surface of the transmitter housing 91. A sensor assembly mounting position is arranged on the lower housing of the transmitter housing 91. The transmitter circuit board assembly 92 includes a transmitter circuit board 921, a Mylar Film 922, a button cell 923, a positive nickel sheet 924 and a negative nickel sheet 925. The Mylar Film 922 is arranged at a bottom end of the transmitter circuit board 921. The button cell 923 is located at a bottom end of the Mylar Film 921. The negative nickel sheet 925 is welded to a negative pad of the transmitter circuit board 921. The positive nickel sheet 924 is welded to a positive pad of the transmitter circuit board 921 and needs to be welded to the button cell 923 by a laser spot welder in advance. The positive nickel sheet 924 and the negative nickel sheet 925 are connected to the button cell 923, and the button cell 923 supplies power to the transmitter circuit board 921. Rigid needle holder holes 95 are formed in the upper housing of the transmitter housing 91 and the transmitter circuit board 921, respectively. A sensor assembly hole 96 is formed in the transmitter bonding adhesive 93. The transmitter circuit board 921 is provided with three electrode contacts 97.

When the transmitter assembly 9 is assembled, first, the transmitter circuit board assembly is assembled; then, the transmitter circuit board assembly is assembled in the transmitter housing 91, and the upper housing and the lower housing of the transmitter housing 91 are welded together by means of an ultrasonic welding machine. After welding, the sealing performance of ultrasonic welding is checked, and then the transmitter bonding adhesive 91 is applied.

A downward indicating arrow is arranged on the side wall of the shell 1 and is adjacent to the push rod sliding hole 17. An indicating groove 312 formed in the needle box bottom shell is located on a same side as the indicating arrow on the side wall of the shell 1. When the needle box is inserted, the direction is determined according to the indicating groove 312 and the indicting arrow.

When the painless patch applicator provided by the present invention is assembled, first, the button 4 is assembled in the Shell A 11. Then, the spiral spring 2 is assembled in the Shell A 11. Next, the turnplate 3 is assembled in the Shell A 11. The turnplate rotating tool is inserted into the rotation hole of the turnplate and properly pulled upwards, and the spiral spring 2 is driven by the turnplate 3 to turn around clockwise twice and is then clamped. The unlocking push rod 5 is assembled in the Shell A 11 and pushed downwards to the maximum extent. Then, the transmitter holder 7 is assembled at the bottom end of the sliding rail 6. The transmitter assembly 9 is assembled in the transmitter holder 7, and whether all buckles are assembled in place is checked. Then, the sliding rail 6/the transmitter holder 7 and the transmitter assembly 9 which are connected are assembled in the Shell A 11 of the patch applicator , and whether all components are assembled in place is checked. Then, the Shell B 12 is clamped on the Shell A 11, and whether all buckles are assembled in place is checked. Finally, the lower portion of the Shell A is covered with the bottom cover 10, and whether all buckles are assembled in place is checked. The whole assembly process is simple, and all parts are assembled without a hot-pressing process.

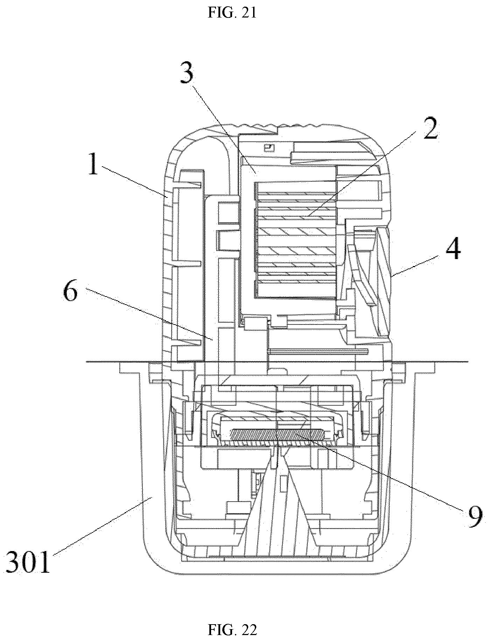

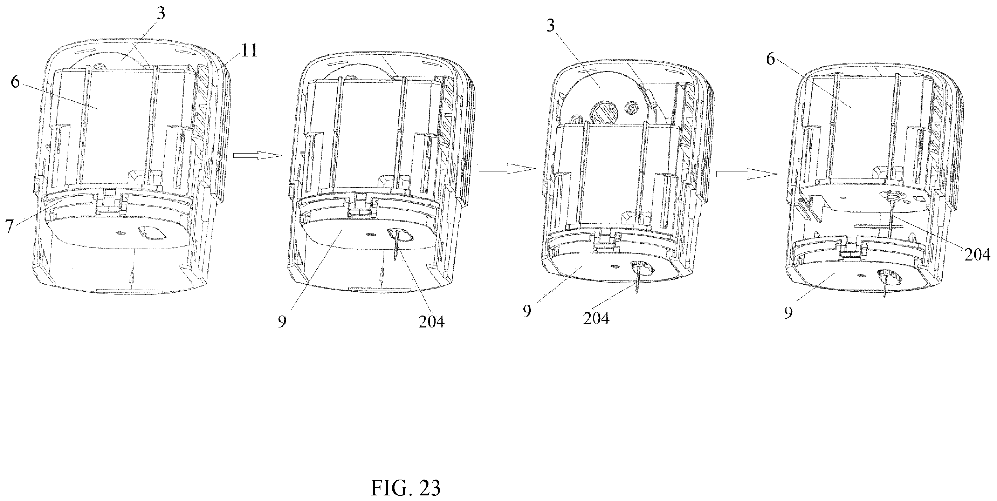

Referring to FIGS. 21, 22 and 23, when the painless patch applicator provided by the present invention is used, the bottom cover 10 is detached, the sealing aluminum foil 303 on the needle box bottom shell 301 is torn away, the needle box bottom shell 301 containing the sensor assembly 200 is mounted on the lower portion of the shell A, the shell 1 is pressed downwards, the painless patch applicator 100 is connected to the sensor assembly 200 in the needle box bottom shell 301, and in the connection process, the sensor assembly 200 penetrates through the sensor assembly hole 96 in the transmitter bonding adhesive 93 and is inserted into the sensor assembly mounting position on the lower housing of the transmitter housing 91, the rigid needle holder 242 sequentially penetrates through the rigid needle holder hole 95 in the transmitter circuit board, the rigid needle holder hole in the upper housing of the transmitter housing 91 and the rigid needle hole 72 in the top end of the transmitter holder 7 and is then clamped in the rigid needle mounting hole 64 in the bottom end of the sliding rail.

At the same time, the push piece 311 on the needle box bottom shell 301 pushes the side push rod insertion piece 53 to slide upwards along the push rod sliding hole 17, the whole unlocking push rod 5 moves upwards, the rear end of the long button pole 42 of the button 4 is separated from the button abutting step 54, the button 4 is unlocked, and the painless patch applicator connected to the sensor assembly is pressed to a suitable position of human skin; then, the button 4 is pressed inwards, the turnplate bump on the ring-shaped peripheral wall of the turnplate 3 is separated from the button bump 41 at the rear end of the long button pole 42, the turnplate 3 is released by the button 4 and driven by precompression potential energy of the spiral spring 2 to rotate, the turnplate stop 34 moves downwards with the rotation of the turnplate 4, the turnplate stop 34, when moving downwards, in the turnplate limiting groove 60 pushes the sliding rail 6 to move downwards to drive the transmitter holder 7 and the rigid needle 204 to move downwards to push the transmitter assembly to move downwards, and the rigid needle 204 drives the vertical implanting part 212 of the sensor electrode 201 to be inserted into the skin; when the spiral spring 2 drives the turnplate 3 to push the sliding rail 6 to the bottom, the rigid needle 204 is completely inserted into the skin, and the needle insertion process is completed.

The vertical implanting part 212 of the sensor electrode 201 is implanted into the skin, the turnplate 3 continues to rotate, the turnplate stop 34 drives the sliding rail 6 to move upwards, and the sliding rail 6 drives the rigid needle 204 to move upwards, such that the rigid needle 204 is withdrawn. Then, the insertion aid of the painless patch applicator is taken down, and the transmitter and the sensor are adhered to the skin by means of the transmitter bonding adhesive 93 at the bottom end of the transmitter assembly. The top ends of the three the conductive silicone elements 205 are in contact with the three electrode contacts 97 on the transmitter circuit board 921 of the transmitter assembly 9, the button battery 26 supplies power to the transmitter circuit board 921, the sensor electrode 201 monitors blood glucose in the human body, and a signal monitored by the transmitter assembly is sent to an external electronic device, such that blood glucose monitoring is realized.

The painless patch applicator provided by the present invention is simpler in structure; the sliding rail is used to replace a link to connect the turnplate and the transmitter holder, the turnplate stop 34 on the turnplate is inserted into the strip-shaped turnplate limiting groove 60 in the sliding rail, and when the turnplate rotates, the turnplate stop 34 is kept in the turnplate limiting groove 60, such that the whole needle insertion process is more stable and reliable; and the button is locked by means of the unlocking push rod to be prevented from being turned on by mistouch of users, such that the painless patch applicator is safer and more practical.

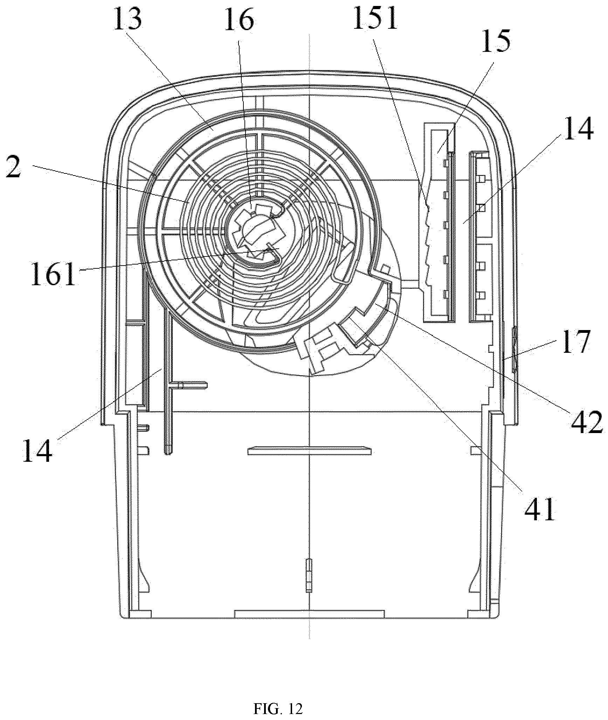

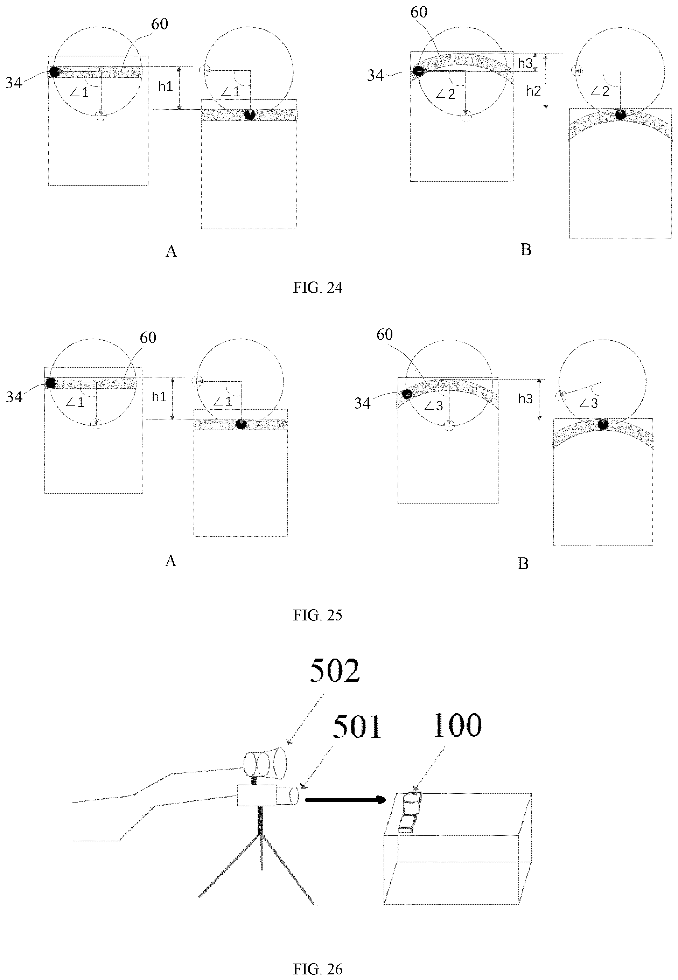

Referring to FIG. 24, the turnplate limiting groove 60 may be in a strip shape or an arc shape that protrudes upwards. A in FIG. 12 illustrates a movement trajectory, in the strip-shaped turnplate limiting groove 60, of the turnplate stop 34 on the turnplate when the sliding rail 6 moves downwards, and B illustrates a movement trajectory, in the arc-shaped turnplate limiting groove 60, of the turnplate stop 34 on the turnplate when the sliding rail 6 moves downwards. Under the same rotation angle of the turnplate ∠1=∠2, the arc-shaped turnplate limiting groove 60 allows for a longer pushing distance of the sliding rail h2>h1.

Referring to FIG. 25, A illustrates a movement trajectory, in the strip-shaped turnplate limiting groove 60, of the turnplate stop 34 on the turnplate when the sliding rail 6 moves downwards, and B illustrates a movement trajectory, in the arc-shaped turnplate limiting groove 60, of the turnplate stop 34 on the turnplate when the sliding rail 6 moves downwards. Under the same sliding distance of the sliding rail h1=h3, the arc-shaped turnplate limiting groove 60 allows for a smaller rotation angle of the turnplate, that is, ∠3<∠1*,* which means that the needle insertion process may be completed within a shorter time.

Referring to FIG. 26, the retention time of the rigid needle in the human body is tested: the retention time in the human body of a rigid needle of a commercially available product and the retention time in the human body of the rigid needle of the painless patch applicator provided by the present invention are detected by means of an OSG030-790UMTZ industrial high-speed camera, specifically as follows:
the patch applicator 100 is connected to the needle box, the sensor and the rigid needle are assembled on the patch applicator 100, the patch applicator 100 is then arranged at a front end of the industrial high-speed camera 501, a camera lens of the industrial high-speed camera 501 is in the same direction as a rigid needle movement region to ensure that the industrial high-speed camera 501 can record the movement process of the rigid needle, and then a fill-in light 501 is mounted to illuminate the insertion aid to provide sufficient light; in the shooting process, the button of the commercially available product and the button of the painless patch applicator provided by the present invention are pressed respectively, and the industrial high-speed camera records the movement process of the commercially available product and the movement process of the painless patch applicator provided by the present invention.

After the shooting process is completed, video images are analyzed by means of software; the number of frames of the video image from the moment the tip of the rigid needle moves outwards to an outlet of the insertion aid to the moment the tip of the rigid needle returns to the outlet of the insertion aid is calculated and taken as the number of frames of the video image of the rigid needle moving outside the insertion aid, and the retention time of the rigid needle is obtained by dividing the number of frames of the video image of the rigid needle moving outside the insertion aid by the movement time of the rigid needle outside the insertion aid obtained based on the average frame rate of the high-speed camera during the test.

For example, when the commercially available product is tested, the average frame rate of the high-speed camera is 2277 fps (2277 frames per second), the average number of frames of the rigid needle moving outside the insertion aid is 110, 110 frames÷2277 fps=48.3 ms, and by calculation, the average movement time of the rigid needle outside the insertion aid is 48.3 ms; and when the painless patch applicator provided by the present invention is tested, the average frame rate of the high-speed camera is 2696 fps (2696 frames per second), the average number of frames of the steel needle moving outside the insertion aid is 12, 12 frames÷2696 fps=4.45 ms, and by calculation, the average movement time of the steel needle outside the insertion aid is 4.45 ms. By multiple tests, the average retention time in the human body of the rigid needle of the painless patch applicator provided by the present invention is 4.45 ms, and the retention time in the human body of the rigid needle of the commercially available insertion aid is 48.3 ms. Compared with the commercially available product, the painless patch applicator provided by the present invention allows for a shorter retention time of the rigid needle in the human body, provides better usage experience for users and relieve paints when the rigid needle is implanted in the sensor; and the whole needle insertion process is faster, and pains are minimized, thereby realizing painless needle insertion. When the sensor fails, the transmitter and the sensor connected to the transmitter are removed.

To sum up, the sensor electrode in the sensor assembly provided by the present invention is directly designed into an L-shaped structure, thereby not being prone to breakage after being implanted into the human body; the sensor electrode is in lateral contact with the the conductive silicone elements, featuring a firm connection, resistance to shaking and good data stability. The needle box provided by the present invention can reliably store the sensor assembly and is convenient to use. The dedicated painless patch applicator provided by the present invention has a few of parts and a simple structure, is easy to assemble, avoids false triggering and makes the whole insertion process more stable and reliable; and needle insertion is realized by one spiral spring, the whole needle insertion process is faster, and pains are minimized, thereby realizing painless needle insertion.

Those ordinarily skilled in the art should appreciate that the above embodiments are merely used for explaining the present invention rather than limiting the present invention, and all modifications and transformations made to the above embodiments within the scope of the essence and spirit of the present invention should fall within the scope of the claims of the present invention.

## Claims

1. A sensor assembly, comprising a sensor electrode, a sensor waterproof soft rubber, a plastic base and a rigid needle, wherein:
the sensor electrode is formed by a horizontal connecting part and a vertical implanting part which are connected to form an L-shaped structure, the horizontal connecting part is provided with three electrodes, and the three electrodes are located on front and back sides of the horizontal connecting parts, respectively;
three conductive silicone mounting holes are formed in a top end of the sensor waterproof soft rubber, the three conductive silicone mounting holes are distributed in a triangular shape, and a conductive silicone is arranged in each of the conductive silicone mounting holes;
a strip-shaped sensor electrode channel is formed inwards in a side wall of the sensor waterproof soft rubber, and the sensor electrode channel is located among the three conductive silicone mounting holes and communicates with the three conductive silicone mounting holes;
the horizontal connecting part of the sensor electrode is inserted into the sensor electrode channel, and the three electrodes on the horizontal connecting part are in interference fit and contact with side surfaces of the three the conductive silicone elements in one-to-one correspondence;
a waterproof soft rubber mounting groove and a vertically through rigid needle hole are formed in the plastic base;
the sensor waterproof soft rubber is arranged in the waterproof soft rubber mounting groove, and the vertical implanting part of the sensor electrode is inserted into the rigid needle hole;
a notch is vertically formed in a side wall of the rigid needle, and the rigid needle penetrates through the rigid needle hole and is disposed around the vertical implanting part of the sensor electrode;
a rigid needle holder is arranged at a top end of the rigid needle, and the rigid needle holder is located above the rigid needle hole.

2. The sensor assembly according to Claim 1, wherein joints between a tip of the rigid needle and two side walls of the notch are arc-shaped.

3. A needle box, comprising a needle box bottom shell, a needle box top shell and the sensor assembly according to Claim 1 or 2;
a push piece is vertically arranged on an inner wall of the needle box bottom shell, and a rigid needle receiving groove is formed in the needle box bottom shell;
the needle box top shell is arranged in the needle box bottom shell, and a vertically through sensor assembly mounting hole is formed in the needle box top shell;
the sensor assembly is arranged in the sensor assembly mounting hole, and a lower portion of the rigid needle of the sensor assembly is inserted into the rigid needle receiving groove.

4. The needle box according to Claim 3, wherein a sealing aluminum foil is heat-sealed over the opening in an upper end of the needle box bottom shell.

5. A dedicated painless patch applicator, used for pushing the sensor assembly in the needle box according to Claim 3 and implanting the vertical implanting part of the sensor electrode of the sensor assembly into human skin, the dedicated painless patch applicator comprising an insertion aid and a transmitter assembly arranged in the insertion aid, wherein the insertion aid comprises a shell, a spiral spring, a turnplate, a button, an unlocking push rod, a sliding rail and a transmitter holder, wherein:
the shell is a hollow cavity structure with an opening in a lower end, formed by a Shell A and a Shell B;
a circular spring mounting groove, a rail sliding groove and a push rod sliding groove are formed in an inner wall of the Shell A, an axial direction of the circular spring mounting groove is perpendicular to an axial direction of the shell, and a stationary shaft is coaxially arranged in the circular spring mounting groove; the rail sliding groove is formed by two vertical rail sliding grooves, and the two vertical rail sliding grooves are arranged on two radial sides of the circular spring mounting groove, respectively; the push rod sliding groove is arranged vertically, and the push rod sliding groove is located between the circular spring mounting groove and one of the vertical rail sliding grooves;
a button mounting groove is formed in an outer wall of the Shell A, and a push rod sliding hole is formed in a side wall of the Shell A;
the spiral spring is mounted in the circular spring mounting groove and disposed around the stationary shaft; the turnplate is rotatably disposed around the stationary shaft, and a ring-shaped peripheral wall of the turnplate is located between the spiral spring and a wall of the circular spring mounting groove; an inner end of the spiral spring is connected to the stationary shaft; an outer end of the spiral spring is connected to the ring-shaped peripheral wall of the turnplate;
a turnplate bump is arranged on an outer surface of the ring-shaped peripheral wall of the turnplate, and a turnplate stop is arranged at a bottom of the turnplate;
the button is movably arranged in the button mounting groove of the Shell A, and a rear end of the button penetrates through a bottom of the button mounting groove to extend into the Shell A; a button bump is arranged on an inner wall of the rear end of the button, and in an initial state, the turnplate bump abuts against the button bump;
a front end of the unlocking push rod is inserted into the push rod sliding groove and slides along the push rod sliding groove; one side of the unlocking push rod is inserted into the push rod sliding hole and slides along the push rod sliding hole, and in the initial state, the rear end of the button abuts against the other side of the unlocking push rod;
a turnplate limiting groove is horizontally formed in a front side of an upper portion of the sliding rail, two sides of the sliding rail are inserted into the two vertical rail sliding grooves in one-to-one correspondence and slide along the corresponding vertical rail sliding grooves, and the turnplate stop is inserted into the turnplate limiting groove;
the transmitter holder is coaxially arranged in the shell;
a top end of the transmitter holder is connected to a bottom end of the sliding rail;
the transmitter assembly is arranged in the transmitter holder;
a rigid needle mounting hole is formed in the bottom end of the sliding rail, and a vertically through rigid needle hole is formed in the top end of the transmitter holder.

6. The dedicated painless patch applicator according to Claim 5, wherein the transmitter assembly comprises a transmitter housing and a transmitter circuit board assembly arranged in the transmitter housing, wherein:
the transmitter housing is formed by an upper housing and a lower housing which are connected together, and a transmitter bonding adhesive is arranged on a bottom surface of the transmitter housing; a sensor assembly mounting position is arranged on the lower housing of the transmitter housing;
the transmitter circuit board comprises a transmitter circuit board, a Mylar Film, a button cell, a positive nickel sheet and a negative nickel sheet, and the Mylar Film is arranged at a bottom end of the transmitter circuit board; the button cell is located at a bottom end of the Mylar Film; the negative nickel sheet is welded to a negative pad of the transmitter circuit board; the positive nickel sheet is welded to a positive pad of the transmitter circuit board, and the positive nickel sheet and the negative nickel sheet are connected to the button cell;
rigid needle holder holes are formed in the upper housing of the transmitter housing and the transmitter circuit board, respectively;
a sensor assembly hole is formed in the transmitter bonding adhesive.

7. The dedicated painless patch applicator according to Claim 6, wherein when the dedicated painless patch applicator is connected to the sensor assembly in the needle box, the sensor assembly penetrates through the sensor assembly hole in the transmitter bonding adhesive and is then inserted into the sensor assembly mounting position on the lower housing of the transmitter housing;
the rigid needle holder sequentially penetrates through the rigid needle holder hole in the transmitter circuit board, the rigid needle holder hole in the upper housing of the transmitter housing and the rigid needle hole in the top end of the transmitter holder and is then clamped in the rigid needle mounting hole in the bottom end of the sliding rail.

8. The dedicated painless patch applicator according to Claim 6, wherein when the dedicated painless patch applicator is connected to the sensor assembly in the needle box, the push piece on the inner wall of the needle box bottom shell pushes the side, inserted into the push rod sliding hole, of the unlocking push rod to slide upwards to drive the whole unlocking push rod to slide upwards.

9. The dedicated painless patch applicator according to Claim 6, wherein a lower portion of the shell is covered with a bottom cover.

10. The dedicated painless patch applicator according to Claim 5, wherein a transmitter holder stop is arranged at a bottom end of the inner wall of the shell, and when the insertion aid is withdrawn after use, the transmitter holder is clamped by the transmitter holder stop.

11. The dedicated painless patch applicator according to Claim 5, wherein a downward indicating arrow is arranged on a side wall of the shell and is adjacent to the push rod sliding hole; and an indicating groove is formed in an upper end of the needle box bottom shell, and the indicating groove is adjacent to the push piece.

12. The dedicated painless patch applicator according to Claim 5, wherein the turn plate limiting groove is strip-shaped or an arc-shaped that protrudes upwards.
